# EUROPEAN PATENT SPECIFICATION

(11) **EP 3 398 774 B1**
(45) Date of publication and mention of the grant of the patent: **15.07.2020**
(21) Application number: 15911785.2
(22) Date of filing: 30.12.2015
(51) Int. Cl.: B29C 64/106, B29C 64/245, B33Y 30/00, B33Y 40/00, A61F 2/06, B29C 64/241, A61F 2/30, B33Y 50/02

(54) **PRINTING MODULE FOR BIOLOGICAL PRINTER, AND BIOLOGICAL PRINTER**
DRUCKMODUL FÜR BIOLOGISCHE DRUCKER UND BIOLOGISCHER DRUCKER
MODULE D'IMPRESSION DE BIO-IMPRIMANTE ET BIO-IMPRIMANTE

(43) Date of publication of application: 07.11.2018
(73) Proprietor: Revotek Co., Ltd, Chengdu, Sichuan 611731 (CN)
(72) Inventor: WEN, Xuemin, Chengdu Sichuan 611731 (CN); LI, Yijun, Chengdu Sichuan 611731 (CN)
(74) Representative: Lavoix
(86) International application number: PCT/CN2015/099795
(87) International publication number: WO 2017/113156

(56) References cited:
- EP-A1- 3 278 765
- WO-A1-2015/023077
- WO-A1-2015/023077
- CN-A- 104 146 794
- CN-A- 105 647 803
- CN-U- 204 723 215
- CN-U- 204 734 579
- CN-U- 204 971 703
- CN-U- 205 295 361

## Description

### FIELD OF THE DISCLOSURE

The present disclosure relates to the technical field of bioprinting, and especially relates to a bioprinter printing assembly and a bioprinter.

### BACKGROUND OF THE DISCLOSURE

3D Biological printing refers to the printing of biological materials (including natural materials, synthetic materials or cellular solutions) into a designed three-dimensional structure through the principles and methods of 3D printing, which is different from 3D printing technology. The biological tissues or organs produced by 3D biological printing technology also have certain biological functions and need to provide conditions for the further growth of cells and tissues. Exactly due to the aforementioned characteristics, the 3D biological printing technology is confronted with many specific technical problems in development.

Among them, in the field of 3D biological printing, the technique of taking cells as a printing material is referred to as cell 3D printing technology. People may utilize cells and biocompatible materials to make bio-ink, so that the nozzle moves and sprays the bio-ink by a program. The bio-ink is printed according to a three-dimensional digital model of the preset target. In the medical field, the 3D biological printing method is commonly used to produce active tissues of the cavity such as artificial blood vessels, which provides an approach for the treatment of many vascular diseases.

The printer for printing a blood vessel in the prior art comprises a rotary rod and a printing spray head. The length and size of the rotary rod are designed according to the shape and size of a blood vessel required to be printed, such that one end of the rotary rod is supported by a bracket and driven by a motor, and the other end is suspended. The printing spray head is located above the rotary rod and is movable relative to a surface of the rotary rod. During the operation, the printing spray head sprays the bio-ink onto the surface of the rotary rod to form a bio-vascular tissue. After the printing is completed, the rotary rod with a vascular tissue is removed and placed into a biological incubator, and various growth factors and differentiation promoting factors are added to accelerate the maturation of the vascular tissue structure and various physiological function, so that a desired blood vessel is obtained.

However, for such solution, within such period of time after the printing spray head sprays the cells onto the rotary rod to form a biological vascular tissue until the entire printing process is over, the cells and the bio-vascular tissue on the rotary rod lack the external nutrient supply, so that both the survival of its cells and the realization of the biological functions will be affected. It can be seen that, for 3D biological printing, nutrient supply is an indispensable and important process, while how to provide a nutrient solution to a carrier on a dynamic rotary rod has always been a technical challenge difficult to be solved in this field. In prior art EP3278765A1, a rotary device for biological printing is provided, which includes a rotary rod and a nutrition supply system, wherein the rotary rod is arranged horizontally and is driven to rotate, the rotary rod has a hollow structure and provided with at least one hole in a surface thereof, such that during a 3D bio-printing process; and the nutrition supply system delivers a nutrition solution to the rotary rod, such that the nutrition solution passes through the hollow structure of the rotary rod and a portion of the nutrition solution exudes via at least one hole in a surface of the rotary rod. In prior art CN204723215U, a blood vessel cast-molding device is provided, which includes a pedestal, a shaft and a group of brackets coaxially connected with the shaft, the shaft is rotatably mounted on the pedestal, the shaft is provided with a hollow cavity extending along the axial direction, the side wall of the shaft is provided with a connecting hole, and nutrient solution enters into the hollow cavity of the shaft through the inlet, and enters into the gaps of adjacent brackets through the connecting hole, so as to realize the cast-molding of blood vessels. In prior art WO2015/023077A1, an apparatus for manufacturing a biodegradable stent is provided, which includes a rotating shaft, a 3D printer and an integrated control device, wherein the 3D printer is provided with a precision multi-axis discharging device which moves so as to spray a biodegradable material on a rotating shaft, and the integrated control device controls the operation of the precision multi-axis discharging device.

### SUMMARY OF THE DISCLOSURE

The object of the present disclosure is to propose a bioprinter printing assembly and a bioprinter, which is capable of supplying an external nutrient solution to the cells and biological tubular tissues on the rotary rod in the printing process.

To achieve the aforementioned object, a first aspect of the present disclosure provides a bioprinter printing assembly according to claim 1.

Furthermore, the bioprinter printing assembly further comprises a sealing device provided between the nutrient solution conveying portion and the rotary rod.

Furthermore, the bioprinter printing assembly further comprises a nutrient solution tank for supplying a nutrient solution to the rotary rod, wherein the nutrient solution tank has a tank port connected with the conveying portion port through a nutrient solution conveying device for controlling the nutrient solution from the nutrient solution tank to into the rotary rod.

Furthermore, the nutrient solution conveying device is further used for adjusting a flow velocity and a flow rate of the nutrient solution; or the bioprinter printing assembly further comprises a nutrient solution controlling device connected with the nutrient solution conveying device in series, and the tank port is connected with the conveying portion port through the nutrient solution conveying device and the nutrient solution control device, wherein the nutrient solution controlling device is used for adjusting a flow velocity and a flow rate of the nutrient solution.

Furthermore, the bioprinter printing assembly further comprises a temperature detection member for detecting a temperature of the nutrient solution or a nutrient solution temperature control device for adjusting a temperature of the nutrient solution.

Further, the nutrient solution temperature control device comprises: a semiconductor cooling pad, a temperature controller and a radiation device, wherein the semiconductor cooling pad is provided at the bottom of the nutrient solution tank to heat or cool the nutrient solution tank under the control of the temperature controller, and the semiconductor cooling pad has a temperature-controlling end which is provided towards the nutrient solution tank and a non-temperature-controlling end which is provided towards the radiation device.

Further, a heat conducting plate or a vapor chamber is provided between the nutrient solution tank and the semiconductor cooling pad.

Further, the rotary rod further comprises an adapter section, on which the nutrient solution supply port is provided, wherein the adapter section is detachably coaxially disposed at an end of the hollow tube section, and the adapter section is rotatably supported on the support body, and the adapter section is drivable to rotate the hollow tube section.

Furthermore, the bioprinter printing assembly further comprises a connector, wherein the adapter section is connected with the hollow tube section through the connector.

Further, the connector is a threaded connector.

Further, the adapter section is internally provided with a hollow portion communicating with the nutrient solution supply port, wherein the hollow portion communicates with the central through hole of the hollow tube section to guide the nutrient solution into the hollow tube section through the hollow portion.

Further, the adapter section is sealingly connected with an end of the hollow tube section.

Furthermore, the bioprinter printing assembly further comprises a servo motor, which is arranged in parallel with the rotary rod, and which is connected to the rotary rod through a synchronous belt pulley and a synchronous belt.

In order to realize the aforementioned object, a second aspect of the present disclosure provides a bioprinter, comprising the printing assembly according to the aforementioned embodiments.

Based on the aforementioned technical solution, the bioprinter printing assembly of the present disclosure, by providing a nutrient solution supply port and a permeable hole on the rotary rod, the external nutrient solution can be guided into the inside of the rotary rod through the nutrient solution supply port in the printing process, and then exudes from the permeable hole to supply the cells and biological tubular tissues on the rotary rod, thereby forming a protection for the cells and the biological tubular tissues, so as to improve the survival rate and biological function of the cells.

### BRIEF DESCRIPTION OF THE ACCOMPANYING DRAWINGS

The drawings described herein are used to provide a further understanding of the present disclosure and constitute a part of the present application. The illustrative embodiments of the present disclosure as well as the descriptions thereof, which are used for explaining the present disclosure, do not constitute improper definitions on the present disclosure. In the drawings:
Figure 1 is a schematic view of the three-dimensional structure of one embodiment of the bioprinter printing assembly of the present disclosure;
Figure 2 is a top view of the bioprinter printing assembly shown in Figure 1;
Figure 3 is an A-A sectional view of the bioprinter printing assembly shown in Figure 2;
Figure 4 is a locally enlarged view of the bioprinter printing assembly shown in Figure 2.

### DETAILED DESCRIPTION OF THE PREFERRED EMBODIMENTS

The present disclosure will be described in detail below. In the following paragraphs, different aspects of the embodiments are defined in more detail. The various aspects thus defined may be combined with any other aspect or aspects unless specifically indicated. In particular, any feature believed to be preferred or advantageous may be considered to a feature believed to be preferred or advantageous with one or more other features.

The terms such as "first" and "second" recited in the present disclosure are only for the convenience of description, so as to distinguish different constituent parts having the same term rather than presenting a sequential or dominant relation.

In the description of the present disclosure, it is necessary to understand that, the azimuth or positional relations indicated by the terms "up", "down", "front", "rear", "left", "right", which are based on the azimuth or positional relations illustrated by the drawings, are only for facilitating description of the present disclosure, rather than indicating or implying that the device referred to has to present a particular azimuth, and be constructed and operated in a particular azimuth, so that it cannot be understood as limiting the protection scope of the present disclosure.

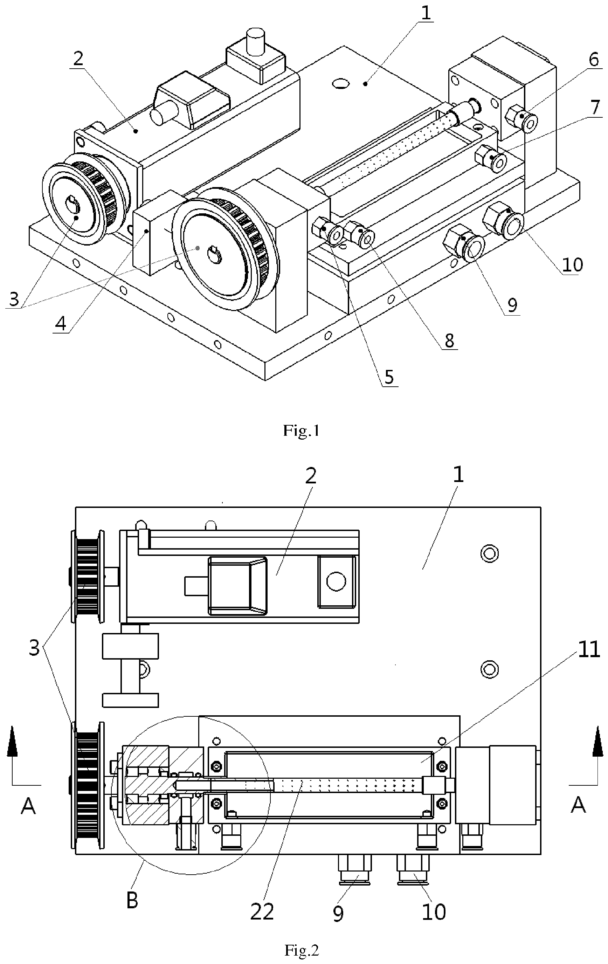

The present disclosure provides a bioprinter printing assembly, which as shown in Figures 1-4 comprises: a support body and a rotary rod provided with a nutrient solution supply port 14, wherein the rotary rod is rotatably supported on the support body, a surface of the rotary rod is used for receiving bio-ink provided by a printing spray head, the rotary rod includes a hollow tube section 22 having a central through hole, and a tube wall of the hollow tube section 22 is provided with a permeable hole 26 communicating with the central through hole, and the nutrient solution supply port communicates with the central through hole of the rotary rod to guide external nutrient solution into the rotary rod.

Moreover, by adjusting the distribution and dimension of the permeable holes 26 in the rotary rod, it is possible to adapt to the requirements of printing the biological tubular tissues of different thicknesses, lengths and natures. For example, the biological tubular tissue may be a blood vessel. In addition, the nutrient solution which may use disposable manner as necessary, can improve the biological safety.

The bioprinter printing assembly of the embodiments of the present disclosure can guide the external nutrient solution into the inside of the rotary rod through the nutrient solution supply port 14 in the printing process, and then exudes from the permeable hole 26 to supply the cells and biological tubular tissues on the rotary rod, thereby forming a protection for the cells and the biological tubular tissues, so as to improve the survival rate and biological function of the cells.

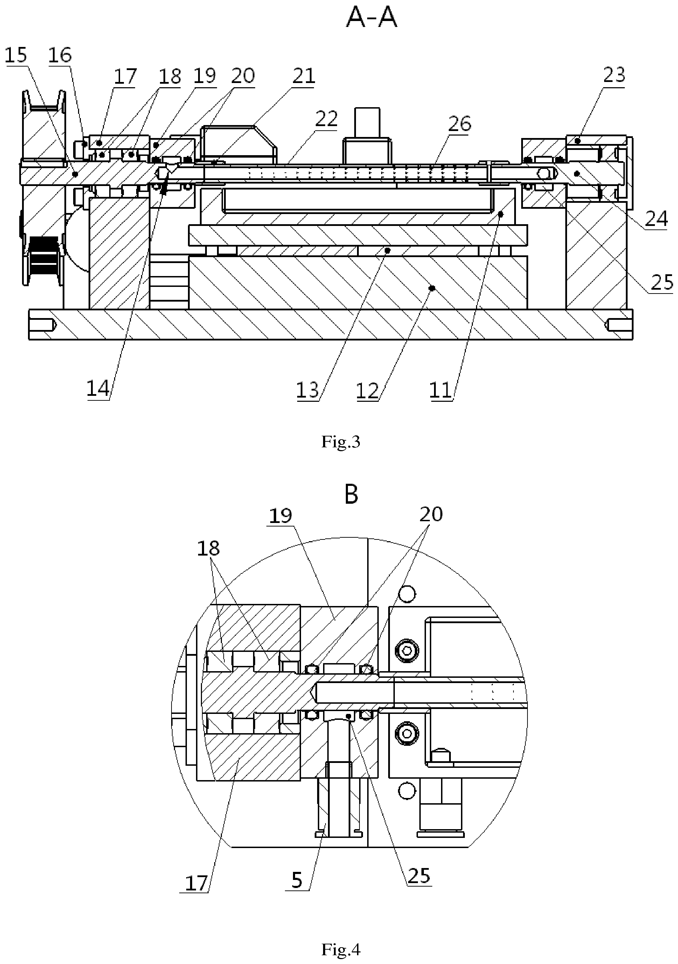

The rotary rod in the embodiment may include the hollow tube section 22 only, or maybe provided with an adapter section only at one end of the hollow tube section 22. The structure shown in Figure 3 may also be adopted. That is, the rotary rod includes a hollow tube section 22 and an adapter section 15 and an adapter section 24 respectively connected at both ends of the hollow tube section 22. The adapter section 15 and the adapter section 24 are a first rotation adapter and a second rotation adapter respectively. Each of the embodiments given below will be described using the rotary rod of the constituent structure shown in Figure 3 as an example.

In order to guide the nutrient solution from the fixed structure to the inside of the rotating rotary rod, at least two types of guiding manners may be used:

First, the nutrient solution may be guided through a conveying tube from an end of the rotary rod. In this case, it is necessary to penetrate a central through hole inside the rotary rod along an axial direction, and necessary to provide dynamic and static adapter joints at the connection between the conveying tube and the rotary rod.

Second, the nutrient solution supply port 14 is provided in a circumferential direction of the rotary rod. Specifically, the rotary rod includes a hollow tube section 22 and an adapter section 15 and an adapter section 24 respectively connected at both ends of the hollow tube section 22. The adapter section 15 and the adapter section 24 are a first rotation adapter and a second rotation adapter, respectively. Correspondingly, the nutrient solution supply port 14 is provided in a circumferential direction of the first rotation adapter and the second rotation adapter, and the two nutrient solution conveying portions 19 are respectively sleeved on the first rotation adapter and the second rotation adapter and have a conveying portion port. Referring to the enlarged view B shown in Figure 4, the nutrient solution conveying section 19 is internally provided with a conversion cavity 25 communicating with the conveying portion port and surrounding the rotary rod in a circumferential direction, for the flow of the nutrient solution. The nutrient solution supply port 14 communicates the central through hole and the conversion cavity 25. For a specific structure, the conversion cavity 25 is opened along its own axis at a middle section of an inner hole of the nutrient solution conveying section 19, and surrounds the corresponding rotation adapter in a circumferential direction. For example, the conversion cavity 25 is a cylindrical cavity.

In order to enable the nutrient solution to flow from the nutrient solution supply port 14 into the central through hole of the hollow tube section 22, the first rotation adapter and the second rotation adapter are both provided with a hollow portion communicating with the nutrient solution supply port 14 from one end proximate to the hollow tube section 22, wherein the hollow portion communicates with the central through hole of the hollow tube section 22 to guide the nutrient solution into the hollow tube section 22 through the hollow portion. For example, the nutrient solution supply port 14 is a hole opened on the outer walls of the first rotation adapter and the second rotation adapter along a radial direction.

The embodiment in which the nutrient solution supply port 14 is provided in a circumferential direction of the rotary rod may make the nutrient solution supply port 14 soaked in the nutrient solution filled up within the conversion cavity 25 at any time, solving the technical challenge of the nutrient solution entering from a fixed structure to a moving structure, and can also store part of the nutrient solution within the conversion cavity 25 in advance so that the supply of the nutrient solution is more stable.

In the embodiment, the support body may include a supporting portion and a nutrient solution conveying portion 19. The first rotation adapter and the second rotation adapter are rotatably supported on the supporting portion. The supporting portion and the nutrient solution conveying portion 19 may be designed to be an independent structure, and may also be designated to be an integrated structure. Here, there is no restriction on a position relation of the supporting portion and the nutrient solution conveying portion 19 along an axis of the rotary rod. In Figure 3, the supporting portion consists in respectively a first support seat 17 and a second support seat 23 fixed on the mounting plate 1. The first support seat 17 supports the first rotation adapter by a bearing 18, and the second support seat 23 supports the second rotation adapter by a bearing. The use of the bilateral support structure improves the rotational stability of the rotary rod.

Further, in order to prevent leakage of the nutrient solution from the gap between the nutrient solution conveying portion 19 and the first rotation adapter or the second rotation adapter, a sealing device, for example a sealing ring 20, which may be specifically an O-type sealing ring, between the nutrient solution conveying portion 19 and the first rotation adapter, and between the nutrient solution conveying portion 19 and the second rotation adapter. Preferably, at least one sealing ring 20 provided on both sides of the conversion cavity 25 may achieve a better sealing effect. The embodiment in which the nutrient solution is guided from a circumferential direction of the adapter section can make the provision of the sealing ring easier. Moreover, a sealed connection should also be made between the adapter section and an end of the hollow tube section 22.

In order to provide a nutrient solution to the biological tubular tissue in the printing process, the bioprinter printing assembly further comprises a nutrient solution tank 11 for supplying a nutrient solution to the rotary rod, wherein the nutrient solution tank 11 has a tank port. In one embodiment, the tank port is connected with the conveying portion port through a nutrient solution conveying device for making the nutrient solution from the nutrient solution tank into the rotary rod smoothly. For example, a pump may be provided between the tank port and the conveying portion port to promote the flow of nutrient solution. Further, the nutrient solution conveying device can also control the flow velocity and the flow rate of the nutrient solution. Specifically, a circulation pump with an adjustable flow rate may be used, for example, a peristaltic pump or a gear pump.

In another embodiment, the bioprinter printing assembly further comprises a nutrient solution controlling device connected with the nutrient solution conveying device in series, and the tank port is connected with the conveying portion port through the nutrient solution conveying device and the nutrient solution control device, wherein the nutrient solution controlling device is used for controlling a flow velocity and a flow rate of the nutrient solution. For example, a circulation pump cooperates with a flow control valve in use.

For such two embodiments, in the printing process, by adjusting the flow velocity and flow of the nutrient solution, the temperature control capability of the rotary rod may also be indirectly adjusted, thus providing a basis for heating and cooling of high temperature printing or substantial printing. In the rotation process of the rotary rod, it is possible to decide whether to open the cycle of the nutrient solution as necessary.

A specific manner of providing various ports on the nutrient solution tank 11 and the nutrient solution conveying portion 19 will be given below. As shown in Figure 1, the conveying portion port on the nutrient solution conveying portion 19 includes: a first pipe joint 5 provided on the nutrient solution conveying portion 19 at a left side (with a direction of Figure 3 as a reference) and a second pipe joint 6 provided on the nutrient solution conveying portion 19 at a right side. The tank port on the nutrient solution tank 11 includes: a third pipe joint 7 and a forth pipe joint 8. The circulation pump may be provided between the first pipe joint 5 and the fourth pipe joint 8, or provided between the second pipe joint 6 and the third pipe joint 7 to promote the flow of the nutrient solution. The flow path of the nutrient solution will be described when a subsequent description is made to the operational principles of the printer, and thus will not be elaborated here. Considering from the convenience and aesthetic effect of the piping layout, the first pipe joint 5, the second pipe joint 6, the third pipe joint 7, the fourth pipe joint 8, and the fifth pipe joint 9 and the sixth pipe joint 10 disposed at a lateral face of the water cooled plate 12 are arranged on the same side of the printer.

In order to ensure the activity of the biological cells in the printing process, the nutrient solution provided needs to be kept within certain temperature range. For this purpose, a temperature detection member for detecting a temperature of the nutrient solution may also be provided. In one implementable embodiment, a temperature detection member is provided at the outlet of the circulation pump for obtaining an outlet temperature of the circulation pump, which is then combined with a cycle time of the nutrient solution such as to enable judging whether the nutrient solution in the rotary rod reaches a required temperature. In addition, the temperature detection device may also be provided at a position such as within the conversion cavity 25 or within the nutrient solution tank 11.

In one embodiment of the present disclosure, the printer further comprises a nutrient solution temperature control device for controlling a temperature of the nutrient solution. As shown in Figure 3, the nutrient solution temperature control device includes: at least one semiconductor cooling pad 13, a temperature controller and a radiation device, wherein the at least one semiconductor cooling pad 13 is provided outside the nutrient solution tank 11, for performing heating or cooling under the control of the temperature controller. The provision of a plurality of semiconductor cooling pads 13 can effectively enhance the cooling or heating power, and can also produce the effect of redundant backup. More preferably, a gap may be provided between two adjacent semiconductor cooling pads 13 to facilitate the installation and wiring of the semiconductor cooling pad 13, and the protective effect can also be produced by placing the wiring within the gap. Wherein, the semiconductor cooling pad 13 has a temperature-controlling end and a non-temperature-controlling end, in which the temperature-controlling end is provided towards the nutrient solution tank 11 to achieve the purpose of controlling the temperature of the nutrient solution tank 11 so as to control the temperature of the nutrient solution within the nutrient solution tank 11, and the non-temperature-controlling end is provided towards the radiation device.

In a specific structural design, the nutrient solution tank 11 is located immediately below the rotary rod, and the radiation device includes a water cooled plate 12. The semiconductor cooling pad 13 is provided below the nutrient solution tank 11 for automatic alteration of an operational mode, including a heating or cooling mode, by a program under the control of an external temperature controller. The water cooled plate 12 is provided below the semiconductor cooling pad 13, and fixed on the mounting plate 1 for taking away the heat produced by the semiconductor cooling pad 13 during operation, wherein the water cooled plate may also be replaced using other radiation means, for example fan cooling.

In order to facilitate the fixation of the semiconductor cooling pad 13, a heat conducting plate or a vapor chamber having a size larger than a bottom area of the tank is designed below the rectangular nutrient solution tank 11. On the one hand, the heat generated by the semiconductor cooling pad 13 may be evenly transferred to the nutrient solution tank 11, and on the other hand, the semiconductor cooling pad 13 may also be sandwiched between the base and the water cooled plate 12, and bolts are provided around the base to achieve the fixation with the water cooled plate 12. A plurality of semiconductor cooling pads 13 may be arranged side by side. For example, since the semiconductor cooling pad 13 is generally square, in order to evenly heat the rectangular nutrient tank 11, two semiconductor cooling pads 13 may be arranged side by side. As shown in Figures 1 and 2, the lateral faces of the water cooled plate 12 are provided with a fifth pipe joint 9 and a sixth pipe joint 10, which are respectively connected to the water inlet and the water outlet of the external water cooled circulation system, for taking away the heat produced by the semiconductor cooling pad 13 during operation.

When heating is required, the temperature control system is in a heating state, at which time the temperature-controlling end of the semiconductor cooling pad 13 is a hot face, and the heat generated by the heating is transferred to the nutrient solution in the nutrient solution tank 11 to achieve the purpose of warming up the nutrient solution. On the contrary, when cooling is required, the temperature-controlling end of the semiconductor cooling pad 13 becomes a cold face to absorb the heat of the nutrient solution in the nutrient solution tank 11 to achieve the purpose of cooling down the nutrient solution. At the same time, the non-temperature-controlling end of the semiconductor becomes a hot face to transfer the heat to the water cooled plate 12, so as to reduce the temperature difference between the hot and cold faces of the semiconductor cooling pad 13 and increase the cooling effect.

The printer in the prior art is further present with another disadvantage. After completion of each printing, it is necessary to remove a support structure of the end together when the rotary rod is removed, so that the removal process is very cumbersome, and it is hard to ensure consistent accuracy of each installation. Moreover, when it is necessary to print biological tubular tissues of different diameters, it is necessary to substitute by a rotary rod of a corresponding diameter, such that the support structure also needs to be re-designed, which makes a poor versatility and adaptability of the printer.

In order to solve such problem, in the improved embodiment of the present disclosure, the rotary rod further includes at least one adapter section in addition to including the hollow tube section 22 (the adapter sections 15 and 24 in Figure 3 are respectively the first rotation adapter and the first rotation adapter). The nutrient solution supply port 14 is provided on the adapter sections 15 and 24, the adapter sections 15 and 24 are detachably coaxially provided at an end of the hollow tube section 22, and rotatably supported on the support body, and are drivable to rotate the hollow tube section 22 so as to cooperate with the printing spray head to effectuate printing the biological tubular tissues or active tissues of other cavities.

The bioprinter printing assembly of the embodiment avoids the structure directly supporting an end of the rotary rod in the prior art by designing the rotary rod into a segmentable and detachable structure and supporting the adapter section by the support body. The rotary rod can be conveniently removed after the printing of the biological tubular tissues is completed, without necessarily removing the support body. Similarly, during the next printing, it may also facilitate mounting the rotary rod to make a very convenient installation and removal, so that it is possible to save the printing time, and easily ensure the installation accuracy of the printer. In addition, when it is necessary to print biological tubular tissues of different diameters, there is only a need to simply replace the port between the hollow tube section and the adapter section, such as to conveniently replace the rotary rods of different specifications to print different biological tubular tissues.

In one structural form, only one adapter section is used. The adapter section is connected to one end of the hollow tube section and is suspended at the other end. When the rotary rod needs to be disassembled and assembled, it is only necessary to disconnect or connect an port portion between the hollow tube section and the adapter section, which does not involve the installation and removal of the support structure.

In another structural form, two adapter sections are used. In the embodiment shown in Figure 3, the adapter section 15 which is the first rotation adapter, and the adapter section 24 which is the second rotation adapter, are detachably provided at both ends of the hollow tube section 22 respectively, and the support body supports the two adapter sections to indirectly effectuate supporting the rotary rod. The rotation of the hollow tube section 22 can be driven by driving one adapter section thereof or by driving two adapter sections at the same time, so as to cooperate with movement of the printing spray head in at least one of the X, Y and Z directions, such that the bio-ink sprayed by the printing spray head is attached to an outer surface of the hollow tube section 22 according to a predetermined trajectory, so as to effectuate printing the biological tubular tissues.

For the embodiment in which two adapter sections are used, since the biological tubular structure takes the rotary rod as a matrix during the printing, and the rotary rod uses such a form as to be supported at both ends, so that the rotation may be very stable, when relatively long biological tubular tissues are printed, it is possible to obtain a high accuracy and reduce the deformation of the rotary rod due to a gravitational effect as much as possible, thereby affecting a predetermined movement trajectory of the bio-ink. After the printing is completed, the hollow tube section 22 may be separated from the adapter section to achieve the removal, and placed into a biological incubator, and various growth factors and differentiation promoting factors are added to accelerate the maturation of the biological tubular tissue structure and various physiological function, so that a desired biological tubular tissue is obtained. When it is necessary to print again, the hollow tube section 22 corresponding to the diameter of the biological tubular tissue is again mounted onto the adapter section.

The following various embodiments will be described by taking a printer using two adapter sections to effectuate printing the biological tubular tissue as an example.

In order to achieve a detachable connection between the hollow tube section 22 and the adapter sections, in one implementation form, referring to Figure 3, the printer further comprises two connectors 21. The first rotation adapter and the second rotation adapter are respectively connected to both ends of the hollow tube section 22 by one connector 21. For example, the connector 21 is a threaded connector, i.e., a threaded hole is opened in the connector 21. The two axial sections of the threaded hole are respectively adapted to the diameters of the rotation adapters and the hollow tube section 22. The threaded hole mated with the rotation adapter or the threaded hole mated with the hollow tube section 22 which has a smaller diameter is provided with an allowance of axial length. Moreover, the connection section of the first rotation adapter and the second rotation adapter with the hollow tube section 22 is provided with an external thread, so that a detachable connection may be realized by the internal thread mated with external thread.

When the hollow tube section 22 needs to be removed upon completion of printing the biological tubular tissue, it is only necessary to rotate two connectors 21. For example, when the threaded hole mated with the rotation adapters is provided with an allowance of axial length, the two connectors 21 move towards both ends respectively so that the hollow tube section 22 may be removed. When it is necessary to print the biological tubular tissue, the hollow tube section 22 is connected between the first rotation adapter and the second rotation adapter, and then the two connectors 21 are rotated. For example, when the threaded hole mated with the rotation adapters is provided with an allowance of axial length, the two connectors 21 are respectively moved towards the middle so that the installation of the hollow tube section 22 may be realized. After the threaded connector is locked, an external rotation force may be transferred to the hollow tube section 22 through the first rotation adapter or the second rotation adapter. It can be seen that the manner of using a threaded connector makes the installation and removal very convenient, without involving the installation and removal of the support structure at both ends, so as to enable ensuring the installation accuracy of the printer, and ensuring that the jumping upon the rotation meets the requirements after the installation of the hollow tube section 22 is completed, so that the trajectory formed by the bio-ink at the surface of the rotary rod is more accurate, so as to ensure that the arrangement of the internal structure of the artificial biological tubular tissue conforms more to the requirements.

In addition, the hollow tube section 22 may choose different specifications of diameter from 1.5 mm to 10 mm according to the purpose of printing different sizes of biological tubular tissues. At the same time, it is also possible to make a series of threaded connectors that match the diameters of the biological tubular tissues. When the biological tubular tissues of different diameters are printed, it may be realized by only replacing the hollow tube section 22 and the threaded connectors on the basis of the printer, without requiring to change a design of the support structure, thereby improving the versatility and operational flexibility of the printer.

In addition to using a threaded connector, those skilled in the art may also use a snap-fit, or ferrule or flange disk type structure to realize a detachable connection.

In another implementation form, the hollow tube section 22 is directly connected to an end of the adapter section. Here, examples will be given for several structural forms to which we may refer. First, if only one adapter section is used, the adapter section and the hollow tube section may be designed into a structure directly connected by threads. Secondly, if two adapter sections are used, a sliding slot may be opened along a radial direction at one end of the first rotation adapter and the second rotation adapter proximate to the hollow tube section. The sliding slot extends to an axial center line of the rotary rod, and both ends of the hollow tube section are provided with an overhang portion, so that it is only necessary to embed the overhang portion from an open end of the sliding slot, and then perform fixation by a pin shaft or other fasteners during the installation.

For each of the aforementioned embodiments, in order to avoid leakage of the nutrient solution during the conveying process, it is best to be sealingly connected the adapter section with an end of the hollow tube section 22. For the embodiment in which the first rotation adapter and the second rotation adapter are connected to the hollow tube section 22 by means of a threaded connector, after the threaded connector is tightened, it is possible to seal a gap between the two rotation adapters and the hollow tube section 22 respectively, and transfer the rotational power to the hollow tube section 22.

In a further embodiment of the present disclosure, the printer drives the rotary rod by the servo motor 2 which moves in conjunction with the external printing spray head under the control of the control system, so as to spirally form a required cavity structure on the hollow tube section 22. The use of the servo motor 2 can very accurately control a rotation velocity of the rotary rod, thereby improving the ability to control the position of the rotary rod, so as to enable controlling a movement in conjunction with the printing spray head.

In some embodiments, in order to save the space, as shown in a top view of Figure 2, the servo motor 2 and the rotary rod are arranged on the mounting plate 1 in parallel. The servo motor 2 and the first rotation adapter are connected through the synchronous belt pulley 3 and the synchronous belt, for transferring the power of the servo motor 2 to the first rotation adapter. The belt transmission manner facilitates the arrangement of the overall structure. Among them, the servo motor 2 is connected with the driving wheel of the synchronous belt pulley 3, and the first rotation adapter is connected with the driven wheel of the synchronous belt pulley 3. For example, a manner such as key connection or fastener connection may be used. The synchronous belt pulley 3 adjusts the tension by the tension adjuster 4, so that the servo motor 2 may very accurately control the position by means of the synchronous belt pulley 3 according to certain velocity ratio. There is no need to perform lubrication for the use of a manner of belt transmission, so that it is possible to avoid that the biological tubular tissues are contaminated by the lubricating oil in the printing process, thereby ensuring the operational safety of the artificial biological tubular tissues. In the case where the contamination by the lubricating oil can be isolated, it is also possible to use a manner of gear transmission or chain transmission.

For the power transfer manner in the embodiment, referring to Figure 3, the first rotation adapter serves as a driving end, and the second rotation adapter serves as a driven end. The first support seat 17 provided at the driving end and the second support seat 23 provided at the driven end are required to ensure that the two nutrient solution conveying portions 19 mounted thereon are coaxial during the installation and debugging, so as to ensure that the jumping of the rotary rod in the rotation process is as slight as possible. Even further, the first rotation adapter is mounted on the first support seat 17 through two bearings 18 so that the supporting force is more stable, and thrusting is performed on both sides by means of the end cap 16 and the nutrient solution conveying portion 19. The connection manner between the driven end and the driving end may use the same form and may be structurally adjusted according to the requirements. In order to achieve a favorable support stiffness, it is best to provide the first support seat 17 and the second support seat 23 outside the two nutrient solution conveying portions 19 so that the support points are as close as possible to an end of the rod. Moreover, all the assemblies related to the rotary rod in the printer are mounted on the same mounting plate 1, which can improve the stability of the overall performance.

In order to make those skilled in the art clearly understand the operational principles of the printer for active tissues of a cavity according to the present disclosure, the working process thereof will be expounded in detail in combination with the specific embodiments shown in Figures 1 to 3.

The nutrient solution is loaded into the nutrient solution tank 11. Set a temperature control temperature, and at the same time turn on the nutrient solution circulation pump. The nutrient solution enters the inlet of the circulation pump from the fourth pipe joint 8, and the pressurized nutrient solution then enters the first pipe joint 5 from the outlet of the circulation pump, so as to flow into a conversion cavity 25 of the nutrient solution conveying portion 19 at a driving end and fill up the conversion cavity 25, then enter a hollow portion of the first rotation adapter by the nutrient solution support port 14, and further flow to the inside of the hollow tube section 22. The nutrient solution may exude outwards by the permeable hole 26 on a tube wall of the hollow tube section 22. The nutrient solution not exuding outwards from the permeable hole 26 further flows to a nutrient solution conveying portion of the driven end, and by undertaking a reversed method of the inflow process, flows out to the second pipe joint 6 which is connected to the third pipe joint 7 on the nutrient solution tank 11, so that the nutrient solution is returned into the nutrient solution tank 11 to maintain an appropriate temperature and follow such cycle. Certainly, it is also possible to choose to make the nutrient solution enter through the second pipe joint 6 to flow out from the first pipe joint 5, and at this time, the conveying direction of the circulation pump also needs to be shifted correspondingly.

By detecting the temperature at the outlet of the external circulation pump as well as the cycle time, it may be inferred whether the nutrient solution in the current rotary rod reaches the set temperature. When the set temperature is reached, under the control of the control system, the servo motor 2 controls the rotation of the rotary rod by means of the synchronous belt pulley 3 so as to move in conjunction with the external printing spray head and spirally form a required cavity structure on the rotary rod. In the printing process, the nutrient solution exudes out of the permeable hole 26 of the rotary rod to provide nutrition to the cells.

In addition, the present disclosure also provides a bioprinter, which comprises the printing assembly described in the aforementioned embodiments. The printing assembly can cooperate with a printing spray head on the printer to effectuate printing biological tubular tissues or active tissues of other cavities. During use, the printing assembly is installed horizontally and placed at 90 degrees with the printing spray head, so that it is possible to effectuate printing transverse biological tubular tissues. The bioprinter, which uses a conveniently detached printing assembly, can improve the operational convenience of the printer and shorten the printing time. Further, since a nutrient solution may be provided to the cells in the printing process, the biological tubular tissues or active tissues of other cavities produced by such printer can maintain a high activity and a long service life.

The above introduces in detail a bioprinter printing assembly and bioprinter provided by the present disclosure. Specific embodiments are applied in this text to elaborate the principles and embodiments of the present disclosure, and the aforementioned descriptions of the embodiments are only used to help understanding the method of the present disclosure.

## Claims

1. A bioprinter printing assembly, comprising:
a support body comprising a supporting portion; and
a rotary rod rotatably supported on the supporting portion of the support body, a surface of the rotary rod is configured to receive bio-ink provided by a printing spray head, **characterized in that** the rotary rod is provided with a nutrient solution supply port (14), wherein
the rotary rod comprises a hollow tube section (22) having a central through hole, and a tube wall of the hollow tube section (22) is provided with a permeable hole (26) communicating with the central through hole, and the nutrient solution supply port (14) communicates with the central through hole of the rotary rod to guide external nutrient solution into the rotary rod, and **in that**
the support body further comprises a nutrient solution conveying portion (19), sleeved on the rotary rod and has a conveying portion port, wherein a conversion cavity (25) is provided in the nutrient solution conveying portion (19) and communicates with the conveying portion port and surrounds the rotary rod in a circumferential direction; wherein the nutrient solution supply port (14) is provided in a circumferential direction of the rotary rod and communicates with the central hole and the conversion cavity (25).

2. The bioprinter printing assembly according to claim 1, further comprising a sealing device provided between the nutrient solution conveying portion (19) and the rotary rod.

3. The bioprinter printing assembly according to claim 1, further comprising a nutrient solution tank (11), for supplying the nutrient solution to the rotary rod, wherein the nutrient solution tank (11) has a tank port connected with the conveying portion port through a nutrient solution conveying device, the nutrient solution conveying device is used for feeding the nutrient solution from the nutrient solution tank (11) into the rotary rod.

4. The bioprinter printing assembly according to claim 3, wherein
the nutrient solution conveying device is configured to adjust a flow velocity and a flow rate of the nutrient solution; or
the bioprinter printing assembly further comprises a nutrient solution controlling device connected with the nutrient solution conveying device in series, and the tank port is connected with the conveying portion port through the nutrient solution conveying device and the nutrient solution control device, wherein the nutrient solution controlling device is configured to adjust a flow velocity and a flow rate of the nutrient solution.

5. The bioprinter printing assembly according to claim 3, further comprising a temperature detection member for detecting a temperature of the nutrient solution or a nutrient solution temperature control device for adjusting a temperature of the nutrient solution.

6. The bioprinter printing assembly according to claim 5, wherein the nutrient solution temperature control device comprises: a semiconductor cooling pad (13), a temperature controller and a radiation device, wherein the semiconductor cooling pad (13) is provided at the bottom of the nutrient solution tank (11) to heat or cool the nutrient solution tank (11) under the control of the temperature controller, and the semiconductor cooling pad (13) has a temperature-controlling end and a non-temperature-controlling end, and the temperature-controlling end is provided towards the nutrient solution tank (11), the non-temperature-controlling end is provided towards the radiation device.

7. The bioprinter printing assembly according to claim 6, wherein a heat conducting plate or a vapor chamber is provided between the nutrient solution tank (11) and the semiconductor cooling pad (13).

8. The bioprinter printing assembly according to claim 1, wherein the rotary rod further comprises an adapter section (15; 24), on which the nutrient solution supply port (14) is provided, the adapter section (15; 24) is detachably coaxially disposed at an end of the hollow tube section (22), and the adapter section (15; 24) is rotatably supported on the support body, and the adapter section (15; 24) is drivable to rotate the hollow tube section (22).

9. The bioprinter printing assembly according to claim 8, further comprising a connector (21), the adapter section (15; 24) is connected with an end of the hollow tube section (22) through the connector (21).

10. The bioprinter printing assembly according to claim 9, wherein the connector (21) is a threaded connector.

11. The bioprinter printing assembly according to claim 8, wherein a hollow portion communicating with the nutrient solution supply port (14) is provided in the adapter section (15; 24)port, wherein the hollow portion communicates with the central through hole of the hollow tube section (22) to supply the nutrient solution into the hollow tube section (22) through the hollow portion.

12. The bioprinter printing assembly according to claim 8, wherein the adapter section (15; 24) is sealingly connected with an end of the hollow tube section (22).

13. The bioprinter printing assembly according to claim 1, further comprising a servo motor (2), which is arranged in parallel with the rotary rod and is connected to the rotary rod through a synchronous belt pulley (3) and a synchronous belt.

14. A bioprinter, comprising the bioprinter printing assembly according to claim 1.

## Patentansprüche

1. Druckmodul eines biologischen Druckers, umfassend:
einen einen Stützabschnitt umfassenden Stützkörper und
einen Drehstab, der auf dem Stützabschnitt des Stützkörpers rotierend getragen wird, wobei eine Oberfläche des Drehstabs derart konfiguriert ist, dass sie eine von einem Druckspritzkopf bereitgestellte biologische Tinte empfängt,
**dadurch gekennzeichnet, dass** der Drehstab mit einem Versorgungsanschluss (14) für eine Nährstofflösung versehen ist, wobei der Drehstab einen hohlen Rohrabschnitt (22) mit einer zentralen Durchgangsöffnung umfasst, und eine Rohrwand des Rohrabschnitts (22) mit einer permeablen Öffnung (26) versehen ist, die mit der zentralen Durchgangsöffnung in Verbindung steht, und der Versorgungsanschluss (14) für die Nährstofflösung mit der zentralen Durchgangsöffnung des Drehstabs in Verbindung steht, um dem Drehstab eine externe Nährstofflösung zuzuführen, und
dass der Stützkörper ferner einen den Drehstab ummantelnden Förderabschnitt (19) für die Nährstofflösung umfasst und einen Förderabschnittsanschluss aufweist, wobei ein Umwandlungshohlraum (25) im Förderabschnitt (19) vorgesehen ist und mit dem Förderabschnittsanschluss in Verbindung steht und den Drehstab in Umfangsrichtung umgibt, wobei der Versorgungsanschluss (14) für die Nährstofflösung in einer Umfangsrichtung des Drehstabs vorgesehen ist und mit der zentralen Öffnung und dem Umwandlungshohlraum (25) in Verbindung steht.

2. Druckmodul nach Anspruch 1, ferner umfassend eine zwischen dem Förderabschnitt (19) für die Nährstofflösung und dem Drehstab vorgesehene Dichtungseinrichtung.

3. Druckmodul nach Anspruch 1, ferner umfassend einen Nährstofflösungstank (11) zur Versorgung des Drehstabs mit der Nährstofflösung, wobei der Tank (11) einen über eine Fördereinrichtung für die Nährstofflösung an den Förderabschnittsanschluss angeschlossenen Tankanschluss aufweist und die Fördereinrichtung dazu verwendet wird, um die Näherstofflösung aus dem Tank (11) dem Drehstab zuzuführen.

4. Druckmodul nach Anspruch 3, wobei
die Fördereinrichtung derart konfiguriert ist, dass sie eine Strömungsgeschwindigkeit und einen Durchfluss der Nährstofflösung einstellt, oder
das Druckmodul ferner eine Nährstofflösungssteuerung umfasst, die mit der Fördereinrichtung reihengeschaltet ist, und der Tankanschluss über die Fördereinrichtung und die Steuerung an den Förderabschnittsanschluss angeschlossen ist, wobei die Steuerung derart konfiguriert ist, dass sie eine Strömungsgeschwindigkeit und einen Durchfluss der Nährstofflösung einstellt.

5. Druckmodul nach Anspruch 3, ferner umfassend ein Temperaturerkennungselement zur Erkennung einer Temperatur der Nährstofflösung oder einen Nährstofflösungstemperaturregler zur Regelung einer Temperatur der Nährstofflösung.

6. Druckmodul nach Anspruch 5, wobei der Temperaturregler umfasst: ein Halbleiterkühlkissen (13), eine Temperatursteuereinheit und ein Bestrahlungsgerät, wobei das Kühlkissen (13) am Boden des Tanks (11) vorgesehen ist, um den Tank (11) durch Steuerung durch die Temperatursteuereinheit zu wärmen bzw. zu kühlen, und das Kühlkissen (13) ein Ende mit Temperaturregelung und ein Ende ohne Temperaturregelung aufweist und das Ende mit Temperaturregelung in der Nähe des Tanks (11) vorgesehen ist und das Ende ohne Temperaturregelung in der Nähe des Bestrahlungsgeräts vorgesehen ist.

7. Druckmodul nach Anspruch 6, wobei eine Wärmeleitplatte oder eine Dampfkammer zwischen dem Tank (11) und dem Kühlkissen (13) vorgesehen ist.

8. Druckmodul nach Anspruch 1, wobei der Drehstab ferner einen Adapterabschnitt (15, 24) umfasst, auf dem der Versorgungsanschluss (14) vorgesehen ist, wobei der Adapterabschnitt (15, 24) an einem Ende des hohlen Rohrabschnitts (22) abnehmbar koaxial angeordnet ist und der Adapterabschnitt (15, 24) auf dem Stützkörper rotierbar getragen wird und der Adapterabschnitt (15, 24) zum Rotieren des Rohrabschnitts (22) angetrieben werden kann.

9. Druckmodul nach Anspruch 8, ferner umfassend einen Verbinder (21), wobei der Förderabschnitt (15; 24) über den Verbinder (21) mit einem Ende des Rohrabschnitts (22) verbunden ist.

10. Druckmodul nach Anspruch 9, wobei der Verbinder (21) ein Verbinder mit Gewinde ist.

11. Druckmodul nach Anspruch 8, wobei ein an den Versorgungsanschluss (14) angeschlossener hohler Abschnitt im Anschluss des Adapterabschnitts (15, 24) vorgesehen ist, wobei der hohle Abschnitt an die zentrale Durchgangsöffnung des hohlen Rohrabschnitts (22) angeschlossen ist, um dem hohlen Rohrabschnitt (22) die Nährstofflösung über den hohlen Abschnitt zuzuführen.

12. Druckmodul nach Anspruch 8, wobei der Adapterabschnitt (15, 24) mit einem Ende des hohlen Rohrabschnitts (22) abdichtend verbunden ist.

13. Druckmodul nach Anspruch 1, ferner umfassend einen Servomotor (2), der parallel zum Drehstab angeordnet ist und über eine Synchronriemenscheibe (3) und einen Synchronriemen an den Drehstab angeschlossen ist.

14. Biologischer Drucker, umfassend das Druckmodul nach Anspruch 1.

## Revendications

1. Ensemble d'impression de bio-imprimante, comprenant :
un corps de support comprenant une partie de support ; et
une tige rotative supportée de manière rotative sur la partie de support du corps de support, une surface de la tige rotative étant configurée pour recevoir une bio-encre fournie par une tête de pulvérisation,
**caractérisé en ce que** la tige rotative est munie d'un orifice d'alimentation en solution nutritive (14), la tige rotative comprenant une section de tube creux (22) ayant un orifice traversant central, et une paroi de tube de la section de tube creux (22) est munie d'un orifice perméable (26) qui communique avec l'orifice traversant central, et l'orifice d'alimentation en solution nutritive (14) communique avec l'orifice traversant central de la tige rotative afin de guider la solution nutritive externe vers la tige rotative,
et **en ce que**
le corps de support comprend en outre une partie d'acheminement de solution nutritive (19), gainée sur la tige rotative, et possède un orifice de partie d'acheminement, une cavité de conversion (25) étant prévue dans la partie d'acheminement de solution nutritive (19), ladite cavité de conversion (25) communiquant avec l'orifice de partie d'acheminement et entourant la tige rotative dans une direction circonférentielle ; l'orifice d'alimentation en solution nutritive (14) étant prévu dans une direction circonférentielle de la tige rotative et communiquant avec l'orifice central et la cavité de conversion (25).

2. Ensemble d'impression de bio-imprimante selon la revendication 1, comprenant en outre un dispositif d'étanchéité prévu entre la partie d'acheminement de solution nutritive (19) et la tige rotative.

3. Ensemble d'impression de bio-imprimante selon la revendication 1, comprenant en outre un réservoir de solution nutritive (11), destiné à fournir la solution nutritive à la tige rotative, dans lequel le réservoir de solution nutritive (11) possède un orifice de réservoir relié à l'orifice de partie d'acheminement par le biais d'un dispositif d'acheminement de solution nutritive, le dispositif d'acheminement de solution nutritive étant utilisé pour fournir la solution nutritive qui provient du réservoir de solution nutritive (11) à la tige rotative.

4. Ensemble d'impression de bio-imprimante selon la revendication 3, dans lequel
le dispositif d'acheminement de solution nutritive est configuré pour ajuster une vitesse d'écoulement et un débit de la solution nutritive ; ou
l'ensemble d'impression de bio-imprimante comprend en outre un dispositif de régulation de solution nutritive relié au dispositif d'acheminement de solution nutritive en série, et l'orifice de réservoir est relié à l'orifice de partie d'acheminement par le biais du dispositif d'acheminement de solution nutritive et du dispositif de régulation de solution nutritive, dans lequel le dispositif de régulation de solution nutritive est configuré pour ajuste rune vitesse d'écoulement et un débit de la solution nutritive.

5. Ensemble d'impression de bio-imprimante selon la revendication 3, comprenant en outre un élément de détection de température destiné à détecter une température de la solution nutritive ou un dispositif de régulation de température de solution nutritive destiné à ajuster une température de la solution nutritive.

6. Ensemble d'impression de bio-imprimante selon la revendication 5, dans lequel le dispositif de régulation de température de solution nutritive comprend : un plot de refroidissement à semi-conducteur (13), un régulateur de température et un dispositif de rayonnement, dans lequel le plot de refroidissement à semi-conducteur (13) est prévu au niveau de la partie inférieure du réservoir de solution nutritive (11) afin de chauffer ou de refroidir le réservoir de solution nutritive (11) sous le contrôle du régulateur de température, et le plot de refroidissement à semi-conducteur (13) possède une extrémité de régulation de température et une extrémité de non-régulation de température, et l'extrémité de régulation de température est prévue vers le réservoir de solution nutritive (11), et l'extrémité de non-régulation de température est prévue vers le dispositif de rayonnement.

7. Ensemble d'impression de bio-imprimante selon la revendication 6, dans lequel une plaque de conduction de chaleur ou une chambre de vapeur est prévue entre le réservoir de solution nutritive (11) et le plot de refroidissement à semi-conducteur (13).

8. Ensemble d'impression de bio-imprimante selon la revendication 1, dans lequel la tige rotative comprend en outre une section d'adaptateur (15 ; 24), sur laquelle l'orifice d'alimentation en solution nutritive (14) est prévu, la section d'adaptateur (15 ; 24) est disposé de manière coaxiale et amovible à une extrémité de la section de tube creux (22), et la section d'adaptateur (15 ; 24) est supportée de manière rotative sur le corps de support, et la section d'adaptateur (15 ; 24) peut être entraînée afin de faire tourner la section de tube creux (22).

9. Ensemble d'impression de bio-imprimante selon la revendication 8, comprenant en outre un connecteur (21), et dans lequel la section d'adaptateur (15 ; 24) est reliée à une extrémité de la section de tube creux (22) par le biais du connecteur (21).

10. Ensemble d'impression de bio-imprimante selon la revendication 9, dans lequel le connecteur (21) est un connecteur fileté.

11. Ensemble d'impression de bio-imprimante selon la revendication 8, dans lequel une partie creuse qui communique avec l'orifice d'alimentation en solution nutritive (14) est prévue dans l'orifice de la section d'adaptateur (15 ; 24), dans lequel la partie creuse communique avec l'orifice traversant central de la section de tube creux (22) afin de fournir la solution nutritive à la section de tube creux (22) par le biais de la partie creuse.

12. Ensemble d'impression de bio-imprimante selon la revendication 8, dans lequel la section d'adaptateur (15 ; 24) est reliée de manière étanche à une extrémité de la section de tube creux (22).

13. Ensemble d'impression de bio-imprimante selon la revendication 1, comprenant en outre un servomoteur (2), qui est prévu en parallèle avec la tige rotative et est relié à la tige rotative par le biais d'une poulie à courroie synchrone (3) et d'une courroie synchrone.

14. Bio-imprimante, comprenant l'ensemble d'impression de bio-imprimante selon la revendication 1.
